# EUROPÄISCHE PATENTSCHRIFT

(11) **EP 1 440 967 B1**
(45) Veröffentlichungstag und Bekanntmachung des Hinweises auf die Patenterteilung: **29.03.2006**
(21) Anmeldenummer: 04010155.2
(22) Anmeldetag: 20.11.1998
(51) Int. Cl.: C07D 213/79, C07B 41/08, C07B 63/02

(54) **Verfahren zur Herstellung von Nicotinsäure**
Process for the preparation of nicotinic acid
Procédé pour la préparation d'acide nicotinique

(30) Priorität: 25.11.1997 CH 271997
(43) Veröffentlichungstag der Anmeldung: 28.07.2004
(62) Teilanmeldung aus: 98122066.8
(73) Patentinhaber: Lonza AG, 3930 Visp (CH)
(72) Erfinder: Chuck, Roderick John, 3902 Brig-Glis (CH); Zacher, Uwe, 3900 Brig (CH)

(56) Entgegenhaltungen:
- DE-A- 2 435 134
- CHEMICAL ABSTRACTS, Bd. 123, Nr. 24, 11. Dezember 1995 (1995-12-11), Columbus, Ohio, US; abstract no.: 317458, FRENKEL, A. V. ET AL: "Thermal decomposition of ammonium salts of nicotinic acid and its analogs in the liquid phase" XP002096346 & CODEN: KHFZAN;ISSN: 0023-1134, KHIM.-FARM.ZH, Bd. 29, Nr. 7, 1995, Seiten 32-36,
- CHEMICAL ABSTRACTS, Bd. 86, Nr. 19, 9. Mai 1977 (1977-05-09), Columbus, Ohio, US; abstract no.: 139772, SUVOROV, B. V. ET AL: "Production of nicotinic acid from coke by-product.beta.-picoline" XP002096347 & KOKS KHIM. (1976), (6), 39-40 CODEN: KOKKAI, 1976,
- CHEMICAL ABSTRACTS, Bd. 86, Nr. 17, 25. April 1977 (1977-04-25), Columbus, Ohio, US; abstract no.: 121171, TRESZCZANOWICZ, EDWARD ET AL: "Nicotinic or isonicotinic acid from 3- or 4-picolines" XP002096348 & PL 75 604 A (INSTYTUT CHEMII PRZEMYSLOWEJ, POL.) 31. Dezember 1974 (1974-12-31)

## Beschreibung

Die vorliegende Erfindung betrifft ein Verfahren zur Herstellung von Nicotinsäure aus Lösungen von Ammoniumnicotinat.

Ein bekanntes Verfahren zur Herstellung von Nicotinsäure beruht auf der Oxidation von 3-Methylpyridin (β-Picolin) mit Luftsauerstoff in Gegenwart von Wasser unter heterogener Katalyse. Dieses Verfahren hat jedoch den Nachteil, dass in einer Nebenreaktion ein Teil des 3-Methylpyridins über eine Folge von Oxidations- und Hydrolyseschritten zu Ammoniak abgebaut wird, welches mit dem Hauptprodukt Nicotinsäure Ammoniumnicotinat bildet. Letzteres ist im Gegensatz zur freien Nicotinsäure gut wasserlöslich und erschwert so die Aufarbeitung des Produktgemischs, ausserdem bedeutet es einen Ausbeuteverlust, wenn es nicht zurück in Nicotinsäure übergeführt werden kann. Letzteres ist beispielsweise durch Zugabe einer starken Säure möglich, wobei jedoch das Ammoniumsalz dieser Säure entsteht, welches abgetrennt und als Abfall entsorgt werden muss. Ausserdem ist hierbei zu beachten, dass Nicotinsäure als Pyridinderivat auch als Base reagieren und mit einem Überschuss der starken Säuren ein Salz bilden kann.

Aufgabe der vorliegenden Erfindung war daher, ein Verfahren bereitzustellen, das die Überführmg von Ammoniumnicotinat in Nicotinsäure ohne Zusatz von Hilfsstoffen und ohne Produktion von Abfällen erlaubt.

Erfindungsgemäss wird diese Aufgabe durch das Verfahren nach Patentanspruch 1 gelöst.

Es ist aus DE-A-24 35 134 bekannt, dass eine wässrige Lösung von Ammoniumnicotinat durch Sprühtrocknung in Nicotinsäure übergeführt werden kann. Hierbei entweicht das Ammoniak zusammen mit dem Wasser im Abgas des Sprühtrockners.
Die nach der Sprühtrocknung erhaltene Nicotinsäure enthält je nach Trocknungstemperatur noch geringe Anteile von Ammoniumnicotinat. Es wurde gefunden, dass durch eine thermische Nachbehandlung in einer Wirbelschicht bei 100 bis 200 °C, vorzugsweise 130 bis 170 °C, diese weiter vermindert werden können. Der hierbei gegebenenfalls entstehende Anteil an staubförmiger Nicotinsäure kann in den Sprühtrockner zurückgeführt werden.

Alternativ zu dieser Nachbehandlung in der Wirbelschicht kann auch eine Nachbehandlung unter vermindertem Druck (Teilvakuum bzw. Vakuum) bei niedrigerer Temperatur durchgeführt werden. Der hierbei angewandte Druck liegt vorteilhaft unter 100 mbar, vorzugsweise unter 50 mbar. Die Temperatur beträgt hierbei zweckmässig 70 bis 150°C, vorzugsweise 80 bis 120°C. Besonders gute Ergebnisse wurden bei 10 bis 15 mbar, 80 bis 90 °C und einer Behandlungsdauer von ½ bis 1 h erzielt. Bei dieser niedrigen Temperatur sind die Verluste durch Sublimation minimal und es wird ein Produkt mit ausgezeichneter Transparenz, also ohne Verfärbung, erhalten.

Die Sprühtrocknung wird vorzugsweise bei einer Trocknungsgastemperatur (am Eintritt) von 160 bis 250 °C durchgeführt. Als Trocknungsgas eignet sich Luft oder ein Inertgas wie Stickstoff oder Argon. Die Austrittstemperatur wird vorteilhaft unter 110 °C gehalten, um eine Sublimation des Produkts zu vermeiden.

Vorzugsweise wird die Sprühtrocknung in einem Fliessbett-Sprühtrockner durchgeführt. Solche Fliessbett-Sprühtrockner sind beispielsweise von der Firma Niro A/S in DK-2860 Söborg/Dänemark unter der Bezeichnung FSD™ erhältlich.

Vorzugsweise wird das erfindungsgemässe Verfahren mit einer Ammoniumnicotinatlösung durchgeführt, die durch Zusatz von Ammoniak zu der gegebenenfalls aufkonzentrierten wässrigen Rohlösung aus der katalytischen Oxidation von 3-Methylpyridin erhalten wurde. Dies kann beispielsweise dadurch geschehen, dass gasfönniges oder wässriges Ammoniak in eine Absorptionskolonne eindosiert wird, welcher das gasförmige Reaktionsgemisch aus dem Oxidationsreaktor zugeführt und als Sumpfprodukt Ammoniumnicotinatlösung sowie über Kopf das überschüssige Wasser abgezogen wird.

Das zur Herstellung der Ammoniumnicotinatlösung benötigte Ammoniak wird hierbei vorzugsweise ganz oder teilweise dem Abgas der Sprühtrocknung entnommen. Hierzu kann beispielsweise das Trocknerabgas unter den Taupunkt gekühlt und das kondensierende Ammoniakwasser abgetrennt und in die Nicotinsäureabsorption zurückgeführt werden. Da, wie bereits erwähnt, bei der Oxidation von 3-Methylpyridin ohnehin geringe Mengen von Ammoniak als Nebenprodukt anfallen, ergibt sich insgesamt ein Ammoniaküberschuss, so dass bei ausreichendem Wirkungsgrad der Ammoniakrückführung im kontinuierlichen Betrieb auf eine Ammoniakzufuhr von aussen ganz verzichtet werden kann.

Ebenso wird das im Abgas der Sprühtrocknung enthaltene Wasser vorzugsweise ganz oder teilweise in den Oxidationsreaktor zurückgeführt. Da bei der Oxidation von 1 mol 3-Methylpyridin zu Nicotinsäure im Idealfall 1 mol Wasser entsteht, ergibt sich ein geringer Überschuss an Wasser, der auf geeignete Weise aus der Anlage auszuschleusen ist. Desgleichen wird etwa vorhandenes unumgesetztes 3-Methylpyridin vorteilhaft in den Oxidationsreaktor rückgeführt. Weiterhin ist es möglich, einer kontinuierlich arbeitenden Anlage zur Durchführung des erfindungsgemässen Verfahrens als Oxidationsmittel im stationären Betrieb anstelle von Luft reinen Sauerstoff zuzuführen und das Trocknungsgas im Kreislauf zu führen, so dass eine Anlage mit minimalem Abgasanfall resultiert.

Ein Vorteil des erfindungsgemässen Verfahrens liegt auch darin, dass die so erhältliche Nicotinsäure ohne zusätzliche Behandlung freifliessend ist und selbst bei hoher Temperatur und Luftfeuchtigkeit kaum zur Verklumpung neigt. Ausserdem kann durch Variation der Betriebsparameter der Sprühtrocknung die Komgrösse des Produkts auf den gewünschten Wert eingestellt werden.

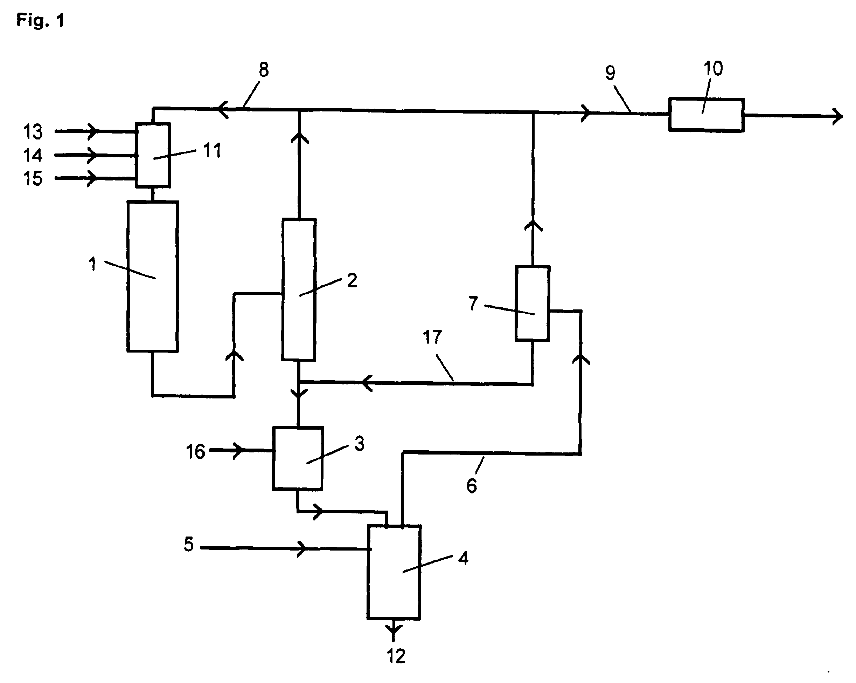

In Figur 1 ist beispielhaft eine zur Durchführung des erfindungsgemässen Verfahrens geeignete kontinuierlich arbeitende Anlage schematisch dargestellt. Es bedeuten im einzelnen:
- 1: Festbettreaktor mit Katalysator
- 2: Absorptionskolonne für teilweise Kondensation des Reaktionsgemisches
- 3: Neutralisation (Ammoniakzugabe)
- 4: Sprühtrockner
- 5: Trocknungsgaszufuhr (Heissluft oder Inertgaskreislauf)
- 6: Abgas vom Sprühtrockner
- 7: Kolonne für teilweise Kondensation von Wasser und Ammoniak
- 8: Gasrückführung zum Reaktor
- 9: Abgas zur Abgasbehandlung
- 10: Abgasbehandlung (Verbrennung)
- 11: Mischer/Vorwärmer
- 12: Produktabnahme (Nicotinsäure)
- 13: 3-Methylpyridin-Einspeisung zum Reaktor
- 14: Sauerstoffeinspeisung (Luft) zum Reaktor
- 15: Wasser(dampf)einspeisung zum Reaktor
- 16: Ammoniakeinspeisung zur Neutralisation
- 17: Ammoniak(wasser)-Rückfuhrung

Die nachfolgenden Beispiele verdeutlichen die Durchführung des erfindungsgemässen Verfahrens, ohne dass darin eine Einschränkung zu sehen ist.

### Beispiel 1

In einem Fliessbett-Sprühtrockner (Niro FSD™-4, Durchmesser 1,2 m, Höhe 2,5 m) wurden 15 l/h einer wässrigen Ammoniumnicotinatlösung (40 Gew.-% Nicotinsäure, 6 Gew.-% Ammoniak) versprüht. Die Eintrittstemperatur des Trocknungsgases (Stickstoff) betrug 220 °C, die Austrittstemperatur 100 °C. Die interne Fliessbettemperatur betrug 70 °C. Es wurde ein freifliessendes Produkt aus 89 Gew.-% Nicotinsäure und 11 Gew.-% Ammoniumnicotinat mit einer Restfeuchte von 0,05 %, einer Schüttdichte von 0,4 kg/l und einer mittleren Teilchengrösse von 451 µm erhalten.

### Beispiel 2

Das gemäss Beispiel 1 erhaltene Nicotinsäure-Sprühgranulat wurde in einem Fliessbett innerhalb von 45 min mit Luft auf maximal 170 °C erhitzt. Das so behandelte Granulat hatte einen Nicotinsäurcgehalt von 99,3 % und eine Schüttdichte von 0,44 kg/l. Es war selbst nach einer Lagerung von 48 h bei 50 °C und 100 % relativer Luftfeuchte noch freifliessend.

### Beispiel 3

Aus dem Kondensat des Reaktionsgemisches der Gasphasenoxidation von 3-Methylpyridin mit Luft in der Gegenwart von Wasser an einem Festbettkatalysator wurde unter Zusatz von Ammoniak eine ca. 30%-ige Lösung von Ammonnikotinat gewonnen. Diese Lösung wurde in einem Labor-Sprühtrockner (Hersteller: Büchi AG, Schweiz) versprüht. Bei einer Eingangsemperatur des Trocknungsgases von 250°C und einer Ausgangstemperatur von 162°C erhielt man bei einem Stickstoffdurchfluss von 600 ml/min Nicotinsäure mit einem Gehalt von 99% bis 100%.
Bei einer Eingangstemperatur von 200 °C und einer Ausgangstemperatur von 130 °C erhielt man bei einem Stickstoffdurchfluss von 600ml/min Nicotinsäure mit einem Gehalt von 96,9%.

## Patentansprüche

1. Verfahren zur Herstellung von Nicotinsäure, **dadurch gekennzeichnet, dass** eine wässrige Lösung von Ammoniumnicotinat sprühgetrocknet und die durch die Sprühtrocknung erhaltene Nicotinsäure einer thermischen Nachbehandlung in einer Wirbelschicht bei 100 bis 200 °C, vorzugsweise 130 bis 170 °C, oder unter vermindertem Druck, vorzugsweise bei weniger als 50 mbar, bei 70 bis 150 °C, vorzugsweise 80 bis 120 °C, unterzogen wird.

2. Verfahren nach Anspruch 1, **dadurch gekennzeichnet, dass** die Sprühtrocknung bei einer Trocknungsgastemperatur von 160 bis 250 °C durchgerührt wird.

3. Verfahren nach Anspruch 1 oder 2, **dadurch gekennzeichnet, dass** die Sprühtrocknung in einem Fliessbett-Sprühtrockner durchgeführt wird.

4. Verfahren nach einem der Ansprüche 1 bis 3, **dadurch gekennzeichnet, dass** als wässrige Lösung von Ammoniumnicotinat die durch Zusatz von Ammoniak zu der gegebenenfalls aufkonzentrierten wässrigen Rohlösung aus der katalytischen Oxidation von 3-Methylpyridin erhaltene Lösung eingesetzt wird.

5. Verfahren nach Anspruch 4, **dadurch gekennzeichnet, dass** das Ammoniak zur Herstellung der Ammoniumnicotinatlösung ganz oder teilweise aus dem Abgas der Sprühtrocknung entnommen wird.

6. Verfahren nach Anspruch 4 oder 5, **dadurch gekennzeichnet, dass** das im Abgas der Sprühtrocknung enthaltene Wasser ganz oder teilweise in den Oxidationsreaktor rückgeführt wird.

## Claims

1. Process for preparing nicotinic acid, **characterized in that** an aqueous solution of ammonium nicotinate is spray-dried and the nicotinic acid obtained by spray-drying is subjected to a thermal post-treatment in a fluidized bed at 100 to 200 °C, preferably at 130 to 170 °C, or under reduced pressure, preferably at less than 50 mbar, at 70 to 150 °C, preferably at 80 to 120 °C.

2. Process according to Claim 1, **characterized in that** the spray-drying is carried out at a drying gas temperature of from 160 to 250 °C.

3. Process according to Claim 1 or 2, **characterized in that** the spray-drying is carried out in a fluidized-bed spray-dryer.

4. Process according to one of Claims 1 to 3, **characterized in that** the aqueous solution of ammonium nicotinate used is the solution produced from the catalytic oxidation of 3-methylpyridine by adding ammonia to the optionally concentrated aqueous crude solution.

5. Process according to Claim 4, **characterized in that** the ammonia for preparing the ammonium nicotinate solution is wholly or partially withdrawn from the spray-drying exhaust gas.

6. Process according to Claim 4 or 5, **characterized in that** the water present in the spray-drying exhaust gas is wholly or partly recycled to the oxidation reactor.

## Revendications

1. Procédé de préparation d'acide nicotinique, **caractérisé en ce qu'**une solution aqueuse de nicotinate d'ammonium est séchée par pulvérisation et l'acide nicotinique obtenu par séchage par pulvérisation est soumis à un post-traitement thermique dans un lit fluidisé à 100 jusqu'à 200 °C, de préférence à 130 jusqu'à 170 °C, ou sous pression réduite, de préférence à moins de 50 mbar, à 70 jusqu'à 150 °C, de préférence à 80 jusqu'à 120 °C.

2. Procédé selon la revendication 1, **caractérisé en ce que** le séchage par pulvérisation est effectué à une température de gaz de séchage de 160 à 250°C.

3. Procédé selon la revendication 1 ou 2, **caractérisé en ce que** le séchage par pulvérisation est effectué dans un sécheur par pulvérisation à lit fluidisé.

4. Procédé selon l'une des revendications 1 à 3, **caractérisé en ce que** la solution aqueuse d'acide nicotinique est obtenue par addition d'ammoniaque à la solution d'acide nicotinique brute aqueuse éventuellement concentrée de l'oxydation catalytique de 3-méthylpyridine.

5. Procédé selon la revendication 4, **caractérisé en ce que** l'ammoniaque pour la préparation de la solution de nicotinate d'ammonium est totalement ou partiellement prélevé des gaz d'échappement du séchage par pulvérisation.

6. Procédé selon la revendication 4 ou 5, **caractérisé en ce que** l'eau contenue dans les gaz d'échappement du séchage par pulvérisation est recyclée totalement ou partiellement dans le réacteur d'oxydation.
